(19)

**Europäisches Patentamt**

**European Patent Office**

**Office européen des brevets**

(11) **EP 4 577 168 B1**

(12) **EUROPEAN PATENT SPECIFICATION**

(45) Date of publication and mention
of the grant of the patent:
**06.05.2026 Bulletin 2026/19**

(21) Application number: **23768438.6**

(22) Date of filing: **17.08.2023**

(51) International Patent Classification (IPC):
*A61F 13/15* (2006.01)    *A41B 9/04* (2006.01)
*A41B 9/12* (2006.01)    *A61F 13/494* (2006.01)
*A61F 13/496* (2006.01)

(52) Cooperative Patent Classification (CPC):
**A61F 13/15268; A41B 9/04; A41B 9/12;**
**A61F 13/15203; A61F 13/49406; A61F 13/496**

(86) International application number:
**PCT/US2023/072407**

(87) International publication number:
**WO 2024/044497 (29.02.2024 Gazette 2024/09)**

(54) **DURABLE ABSORBENT PANT ADAPTED FOR USE WITH A REMOVABLE ABSORBENT INSERT**

HALTBARE ABSORBIERENDE HOSE ZUR VERWENDUNG MIT EINEM ENTFERNBAREN
ABSORBIERENDEN EINSATZ

CULOTTE ABSORBANTE DURABLE CONÇUE POUR ÊTRE UTILISÉE AVEC UN INSERT
ABSORBANT AMOVIBLE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **25.08.2022  US 202263400918 P**

(43) Date of publication of application:
**02.07.2025  Bulletin 2025/27**

(73) Proprietor: **The Procter & Gamble Company
Cincinnati, OH 45202 (US)**

(72) Inventor: **STANLEY, Scott, Kendyl
Cincinnati, Ohio 45202 (US)**

(74) Representative: **P&G Patent Germany
Procter & Gamble Service GmbH
Sulzbacher Straße 40
65824 Schwalbach am Taunus (DE)**

(56) References cited:
**CA-A1- 2 255 465        US-A1- 2014 257 229
US-A1- 2021 100 698        US-A1- 2021 290 447**

**Description**

Field of the Invention

This disclosure relates to durable absorbent pants adapted for use with removable absorbent inserts and designed to contain and absorb menstrual fluids.

Background

A variety of absorbent products, including disposable absorbent products as well as durable, reusable absorbent products, are commercially available for absorbing and retaining urine, menstrual fluids, and other vaginal discharges. In particular, the popularity of durable, reusable absorbent products, is growing. Many consumers may prefer the fit and feel of durable, reusable absorbent products, which tend to feel more like underwear. Systems comprising a washable and reusable product, such as a durable menstrual short or pant, in combination with a disposable absorbent pad are also known. In particular, a system comprising a washable and reusable pant that includes a fabric channel or casing, which has two open ends, for holding a disposable pad is known.

Prior art is known from US2021/100698 and US2021/290447.

However, the existing products and systems have a number of disadvantages. For example, with regard to the system comprising a reusable pant that includes a fabric channel for holding a disposable pad, the two open ends of the channel may allow the disposable pad to shift during use. Also, the presence of two open ends may cause confusion for a user as to the proper insertion orientation of the pad. Some existing products and systems provide insufficient capacity and/or fluid handling performance for all day wear or for users that have heavy insults of urine or menstrual fluid and/or may leak, due to poor fit or improper placement, resulting in soiling of a wearer's clothing or bedding. Therefore, there is a need for an absorbent product or system that provides improved capacity and/or fluid handling to enable all-day wear, without leakage, while delivering dryness and freshness renewal throughout the all-day wear.

SUMMARY OF THE INVENTION

The present invention relates to a durable absorbent pant adapted to accommodate one or more removable absorbent inserts, comprising: a front waist portion with a front waist edge and left and right front leg opening edges; a rear waist portion with a rear waist edge and left and right rear leg opening edges; a crotch portion comprising a gusset, the crotch portion having a forward portion meeting the front waist portion and a rearward portion meeting the rear waist portion; and left and right hip side portions joining the front waist portion to the rear waist portion and thereby forming a waist opening with a waist opening edge comprising the front waist edge and the rear waist edge, and left and right leg openings; where each of the front waist portion, rear waist portion and gusset comprises a knitted or woven material; where the gusset comprises a durable absorbent panel comprising a wearer-facing, liquid permeable top layer, a durable absorbent layer subjacent to the wearer-facing liquid permeable top layer, a liquid impermeable barrier layer subjacent to the durable absorbent layer, the wearer-facing liquid permeable top layer and the durable absorbent layer forming an enveloping structure having a closed end and an open end adapted to receive the one or more removable absorbent inserts in a position subjacent to the wearer-facing, liquid permeable top layer of the durable absorbent panel.

The present disclosure also relates to an array of packages comprising two or more different sizes of durable absorbent pants adapted to accommodate one or more removable absorbent inserts, the array comprising: a. a first package and a second package, where the first package comprises a first durable absorbent pant being a first size and the second package comprises a second durable absorbent pant being a second size, where the second size is different than the first size, where each of the first and second durable absorbent pants comprises: i. a front waist portion with a front waist edge and left and right front leg opening edges; ii. a rear waist portion with a rear waist edge and left and right rear leg opening edges; iii. a crotch portion comprising a gusset, the crotch portion having a forward portion meeting the front waist portion and a rearward portion meeting the rear waist portion; and iv. left and right hip side portions joining the front waist portion to the rear waist portion and thereby forming a waist opening with a waist opening edge comprising the front waist edge and the rear waist edge, and left and right leg openings; where each of the front waist portion, rear waist portion and gusset comprises a knitted or woven material; where the gusset comprises a durable absorbent panel comprising a wearer-facing, liquid permeable top layer, a durable absorbent layer subjacent to the wearer-facing liquid permeable top layer, a liquid impermeable barrier layer subjacent to the durable absorbent layer, the wearer-facing liquid permeable top layer and the durable absorbent layer forming an enveloping structure having a closed end and an open end adapted to receive the one or more removable absorbent inserts in a position subjacent to the wearer-facing, liquid permeable top layer of the durable absorbent panel.

The present disclosure also relates to an array of packages comprising a durable absorbent pant adapted to accommodate one or more removable absorbent inserts, the array comprising: a. a first package, where the first package comprises a durable absorbent pant comprising: i. a front waist portion with a front waist edge and left and right front leg opening edges; ii. a rear waist portion with a rear waist edge and left and right rear leg opening edges; iii. a crotch portion comprising a gusset, the crotch portion having a forward portion meeting the front waist portion and a rearward portion meeting the rear waist portion; and iv. left and right hip side portions joining the front waist portion to the rear waist portion and thereby forming a waist opening with a waist opening edge comprising the front waist edge and the rear waist edge, and left and right leg openings; where each of the front waist portion, rear waist portion and gusset comprises a knitted or woven material; where the gusset comprises a durable absorbent panel comprising a wearer-facing, liquid permeable top layer, a durable absorbent layer subjacent to the wearer-facing liquid permeable top layer, a liquid impermeable barrier layer subjacent to the durable absorbent layer, the wearer-facing liquid permeable top layer and the durable absorbent layer forming an enveloping structure having a closed end and an open end adapted to receive the one or more removable absorbent inserts in a position subjacent to the wearer-facing, liquid permeable top layer of the durable absorbent panel; b. a second package, where the second package comprises a first removable absorbent insert having a first capacity and configured for insertion into the durable absorbent pant; c. a third package, where the third package comprises a second removable absorbent insert having a second capacity and configured for insertion into the durable absorbent pant; where the length and/or width of the first removable absorbent insert is about the same as the length and/or width of the second removable absorbent insert.

Brief Description of the Drawings

FIG. 1 is a simplified depiction of an example of a durable absorbent brief pant, as it would appear laid out flat on a horizontal planar surface, front waist portion facing up.

FIG. 2A is a depiction of an example of the brief pant of FIG. 1, in an opened configuration where the front and rear waist portions have been separated at the hip portions or hip side seams, as it would appear laid out flat on a horizontal planar surface, wearer-facing surfaces facing up.

FIG. 2B is a depiction of another example of the brief pant of FIG. 1, in an opened configuration where the front and rear waist portions have been separated at the hip portions or hip side seams, as it would appear laid out flat on a horizontal planar surface, wearer-facing surfaces facing up.

FIGS. 3A-3E are schematic longitudinal cross sections of various alternative examples of a brief pant as depicted in FIG. 2, taken along line 3-3 in FIG. 2A.

FIG. 4A is a simplified depiction of an example of a brief pant, as it would appear laid out flat on a horizontal planar surface, front waist portion facing up.

FIG. 4B is a simplified depiction of an example of a durable absorbent shorts pant (or legged pant), as it would appear laid out flat on a horizontal planar surface, front waist portion facing up.

FIG. 5A is a schematic lateral cross section of an example of a durable absorbent brief pant as depicted in FIG. 2A, taken along line 5-5 in FIG. 2A.

FIG. 5B is a schematic lateral cross section of an example of a durable absorbent brief pant as depicted in FIG. 2B, taken along line 5-5 in FIG. 2B.

FIG. 6 is a plan view of an example of a crotch gusset laid out on a horizontal surface, wearer-facing surface facing the viewer.

Definitions

"Array of products" means first and second structurally differing products concurrently offered for sale and being associated with a common trademark, each product bearing, or being associated with packaging that includes, information that informs a purchaser of either product that the first product is designed for use in combination with the second product, or vice versa.

With respect to a durable absorbent pant in an opened configuration, laid out flat on a horizontal planar surface, "longitudinal" refers to a direction generally perpendicular to a line tangent each of the left and right leg opening edges where they are closest the front waist edge. With respect to a durable absorbent pant in an assembled configuration, laid out flat on a horizontal planar surface, front waist portion facing up, "longitudinal" refers to a direction generally perpendicular to a line tangent each of the left and right leg opening edges where they are closest the front waist edge. "Length" refers to a dimension measured along the longitudinal direction.

With respect to a durable absorbent pant in an opened configuration, laid out flat on a horizontal planar surface, "lateral" refers to a direction generally parallel to a line tangent each of the left and right leg opening edges where they are closest the front waist edge. With respect to a durable absorbent pant in an assembled configuration, laid out flat on a horizontal planar surface, front waist portion facing up, "lateral" refers to a direction generally parallel to a line tangent each of the left and right leg opening edges where they are closest the front waist edge. "Width" refers to a dimension measured along the lateral direction.

With respect to a wearable garment such as a pant, "durable" means made predominately (as a percentage or fraction of its outward-facing surface area) of cloth material that is knitted and/or woven from natural, semi-synthetic or synthetic fiber, thread or yarn, and which may be normally laundered or hand-washed and dried for reuse/re-wear a plurality of times without substantial destruction thereto. Furthermore, the durable absorbent pant or component of the present invention is not a disposable absorbent article.

A yarn, thread, fiber, filament, web, film or fabric material, or a laminate or composite of any of these, is considered to be "elastic" or "elastomeric" for purposes herein if, when a tensile force no greater than 50 gf/mm (gf per mm of sample width, where width is measured perpendicular to the stretch direction) is applied to the subject material along a stretch direction, the material may be extended along the direction to an elongated dimension of at least 130% of its original relaxed dimension (i.e., can extend at least 30%), without rupture or breakage which substantially damages the subject material; and when the force is removed from the subject material, the material retracts along the stretch direction to recover at least 40% of such elongation. To illustrate, if a section of fabric having an original relaxed length of 100 mm and a width of 40 mm can be elongated by tensile force of 2000 gf (50 gf/mm) in a direction along its length to 130 mm length without substantial damage, and will retract upon removal of the force to a length no greater than 118 mm (130 mm - 118 mm = 12 mm = 40% of 30 mm), it is "elastic" as defined herein. "Elongation," used herein to quantify and express an amount of strain imparted to an elastic material in a stretch direction, means: {[(strained length of the strand) - (length of the strand before straining)] / (length of the strand before straining)}, $\times$ 100%.

With respect to two opposing surfaces of a layer component of a pant, "wearer-facing" refers to the surface that faces the wearer's skin when the pant is worn normally; and "outward-facing" refers to the surface that faces away from the wearer's skin. With respect to two distinct layered components of a pant, the "wearer-facing" component is the component that is disposed closer to the wearer's skin when the pant is worn normally; and the "outward-facing" component is the component that is disposed farther from the wearer's skin.

For purposes herein, a crotch gusset has a "liquid impermeable barrier layer" if it exhibits z-Direction Leakage no greater than 0.1 ml of test fluid into a section of filter paper, in the Liquid Impermeability test described below.

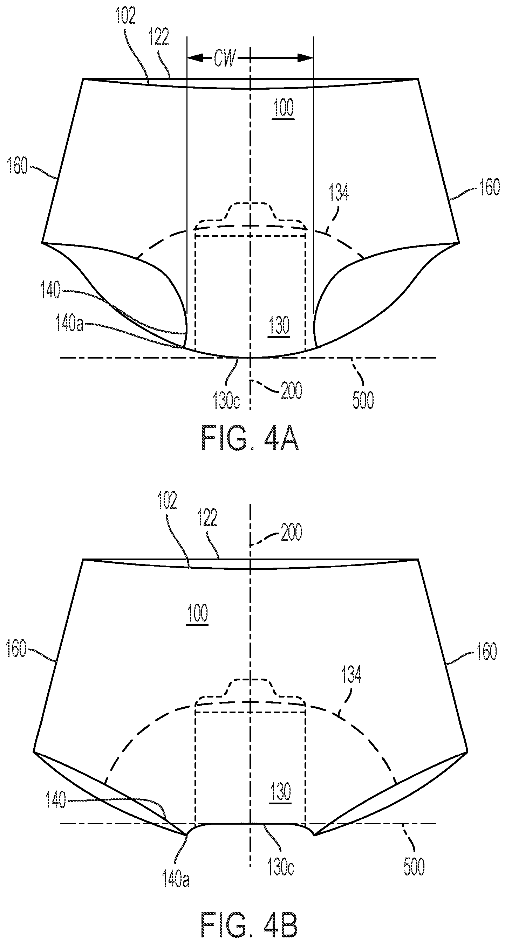

For purposes herein, a "brief pant" is distinguished from a "shorts pant" in an underwear garment by the configuration of the leg edges with respect to the crotch portion, resulting from the manner in which the component materials are shaped, sized, proportioned and seamed or otherwise affixed together. FIG. 4A depicts an example of a brief pant and FIG. 4B depicts an example of a shorts pant. When the garment in its assembled condition is laid out flat on a horizontal planar surface, front waist portion facing up, and a lateral crotch tangent line 500 is drawn perpendicularly to longitudinal axis 200 and tangent to the point at which the crotch portion lower profile 130c intersects the longitudinal axis 200, for a brief pant, the lowermost points 140a along the crotch leg opening edges 140 are disposed along or above the crotch tangent line 500 (i.e., toward the front waist edge 102) (see FIG. 4A); and for a shorts pant, the lowermost points 140a along the crotch leg opening edges 140 are disposed below the crotch tangent line 500 (i.e., away from the front waist edge 102) (see FIG. 4B).

With respect to a durable absorbent pant in an opened configuration, laid out flat on a horizontal planar surface, the "z-direction" is the direction orthogonal to the longitudinal and lateral directions, i.e., the vertical direction relative the horizontal planar surface.

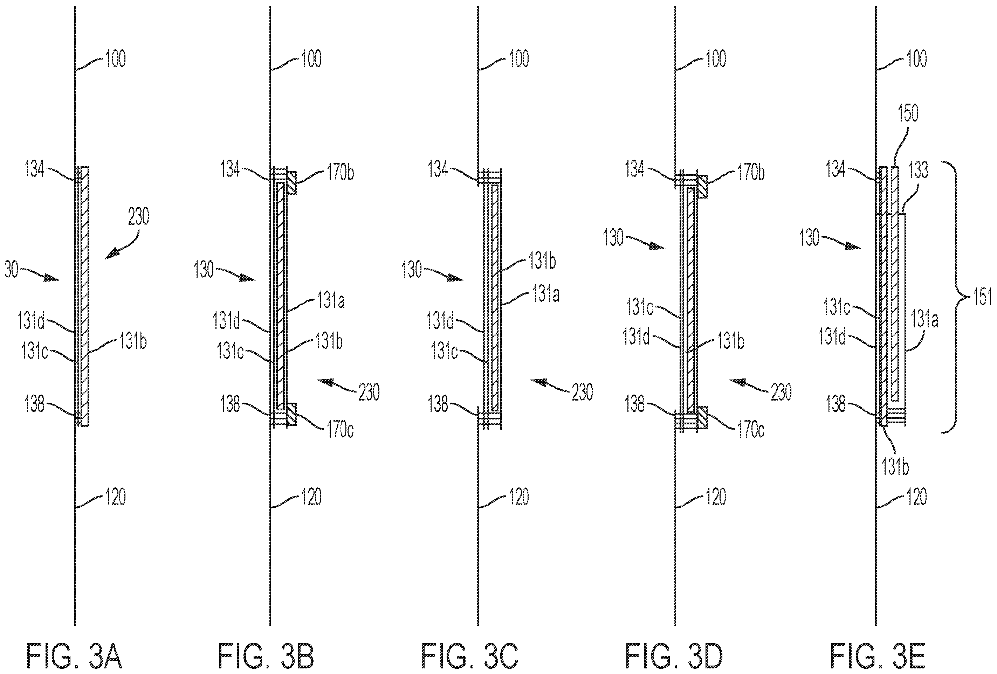

With respect to respective layer components in a crotch gusset of a durable absorbent pant in an opened configuration, laid out flat on a horizontal planar surface, wearer-facing surfaces facing up, as between first and second layer components in the crotch portion, the terms "above," "superadjacent," "below," "subjacent" and/or "beneath" describe the components' disposition along the z-direction relative each other. Thus, for example, referring to FIG. 3E, a wearer-facing, liquid permeable top layer 131a is disposed "above" an outward-facing, liquid impermeable barrier layer 131c, and conversely, the outward-facing, liquid impermeable barrier layer 131c is disposed "below" the wearer-facing, liquid permeable top layer 131a and/or the durable absorbent layer 131b. "Superadjacent" and "subjacent" with respect to two layer components, mean further that the two layer components are disposed in direct surface-to-surface contact with each other.

Description

As noted in the Background, systems comprising a washable and reusable product, such as a menstrual short or pant, in combination with a disposable absorbent pad are known. However, the existing products and systems have a number of disadvantages. For example, the capacity and/or fluid handling performance of existing products and systems is generally insufficient for all day wear or for users that have heavy insults of urine or menstrual fluid. Furthermore, existing products and systems may leak, due to poor fit or improper placement, resulting in soiling of a wearer's clothing or bedding. It has been learned, however, that an absorbent product or system that provides improved capacity and/or fluid handling to enable all day wear, without leakage, while delivering dryness and freshness renewal throughout the all-day wear, may be designed.

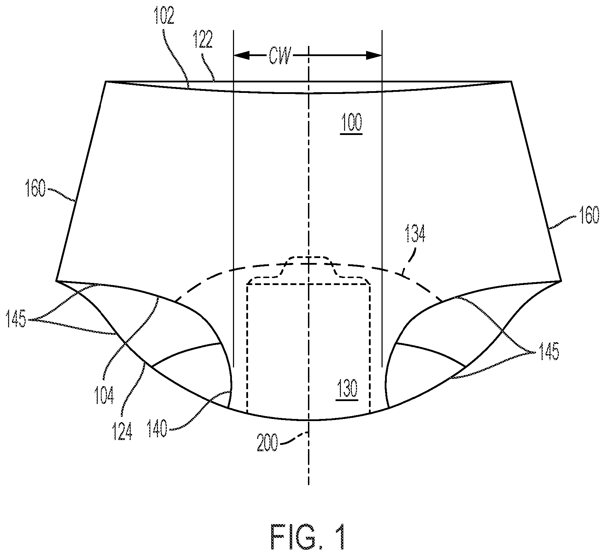

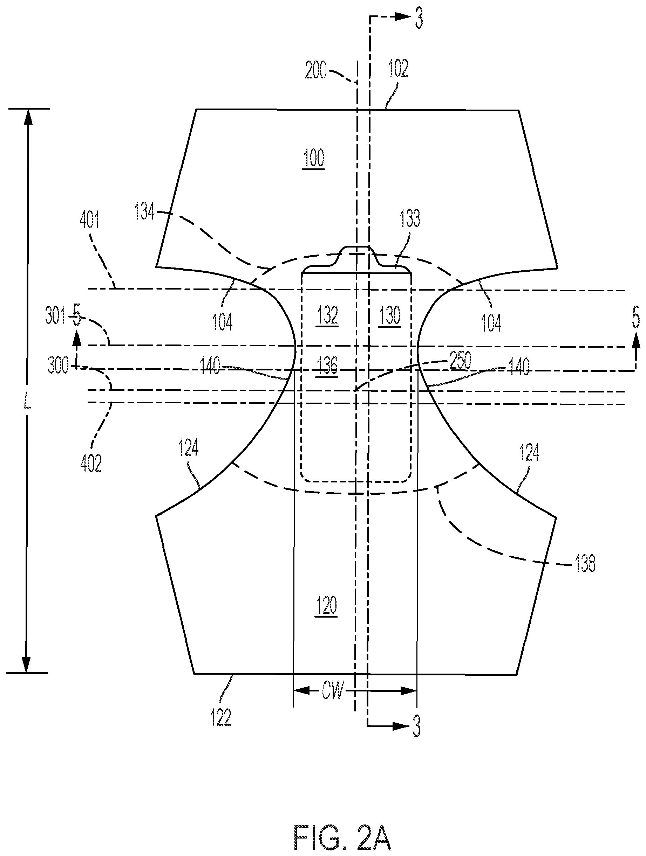

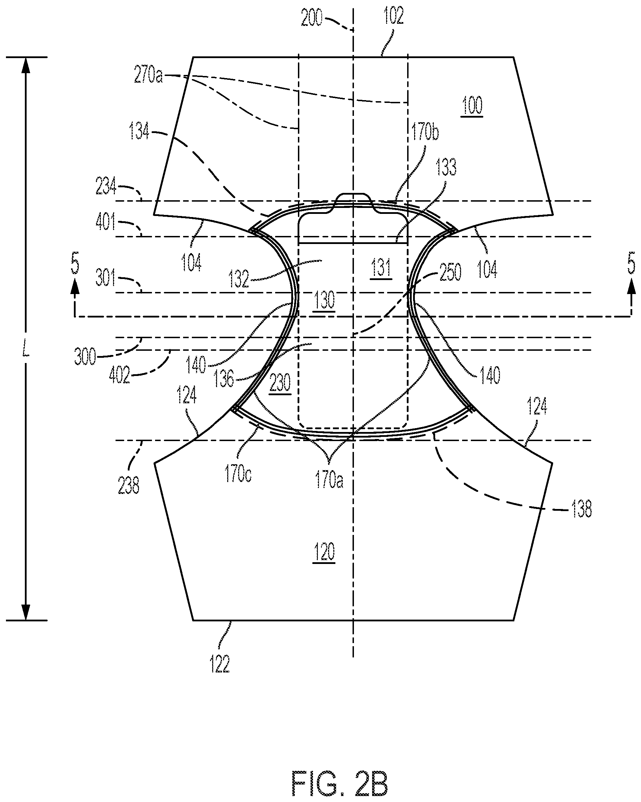

Referring to FIGS. 1 and 2, a durable absorbent brief pant may include a front waist portion 100, a rear waist portion 120 and a crotch portion 130 bridging the front and rear waist portions. Front waist portion 100 has a front waist edge 102, and left and right front leg opening edges 104. Rear waist portion 120 has a rear waist edge 122, and left and right rear leg opening edges 124. Crotch portion 130 has left and right crotch leg opening edges 140. Crotch portion 130 may include or consist of a crotch gusset 230 that may include several layer components that will be described further below. For purposes herein, the "crotch portion 130" is a portion of a pant identified as described herein, independently of specific components or structures. A "crotch gusset 230" is structural component that comprises a durable absorbent panel that includes at least two distinct layers including a durable absorbent layer and a liquid impermeable barrier layer, and bridges the front waist portion 100 and the rear waist portion 120.

The crotch gusset may include a durable absorbent panel comprising multiple layers, e.g., wearer-facing, liquid permeable top layer 131a, durable absorbent layer 131b, outward facing, liquid impermeable barrier layer 131c, and an optional outer fabric layer 131d, that are joined to each other by any suitable mechanism at forward and rearward seams 134, 138 and crotch side seams 135 proximate the leg edges 140. The joining or bonding mechanism may be a system of stitching to affix the layers together; however, for purposes of liquid containment it may be desired that the joining mechanism include a generally hydrophobic, water insoluble adhesive, by itself or as a supplement to stitching.

The liquid permeable top layer 131a preferably comprises a mesh layer with stretch and softness, such as a power mesh fabric that is 90% nylon and 10% spandex. One or more mesh layers may be used to promote a fresh feeling for a user and to sufficiently cover the underlying absorbent core and/or limit the visibility of the one or more removable absorbent inserts (when present). Additionally, color matching may be used with respect to the liquid permeable top layer 131a, one or more of the inserts 150, and/or the durable absorbent layer 131b, to give a more underwear-like appearance. Additionally, one or more spacer fabrics may be used as a liquid permeable top layer 131a and/or durable absorbent layer 131b. A spacer fabric is a three-dimensional knitted fabric consisting of two or more separate knitted substrates, which are joined together or kept apart by spacer yarns or a layer of pile threads.

Turning to the liquid impermeable barrier layer 131c, the liquid impermeable barrier layer 131c (FIGs. 3D and 3E) prevents absorbed urine or menses from passing from the durable absorbent layer 131b to an outer fabric layer 131d or even to outer clothing. In some examples, a barrier layer may be formed of or include a suitable liquid impermeable polymer film. In some examples, the film composition may be selected to have elastic elongation capability, providing suitable elastic stretch attributes to the combination of layers present in the crotch portion 130 and/or crotch gusset 230. In some examples, an outward-facing, liquid impermeable barrier layer 131c may comprise a film formed in whole or in part of a polyurethane- or polyester-based resin. In other examples, an outward-facing, liquid impermeable barrier layer 131c may comprise a film that is formed by extrusion or other application of thermoplastic film resin in molten or semi-molten form directly onto the outward-facing surface of an overlying layer, such as the durable absorbent layer 131b, such that the film resin while still molten partially penetrates the fabric and thereby forms a liquid impermeable film that is partially mechanically enmeshed in and/or made integral with the fabric. This also has the effects of consolidating layers, which can reduce caliper in the crotch portion 130, and reducing or preventing wrinkling or bunching of the durable absorbent layer 131b upon elastic contraction. In a particular example, a polyurethane or polyester film may be formed by extrusion or other application of molten thermoplastic resin directly onto an outward-facing surface of a fabric that serves as or forms a component of the durable absorbent layer 131b.

The material selected for the barrier layer may also be vapor permeable or "breathable" in that it can permit gas or water vapor to pass therethrough, while still being effectively liquid impermeable under ordinary conditions of the use contemplated herein, via a combination of having a porous structure for vapor permeability, but sufficiently small pore sizes and surfaces having low wettability (e.g. hydrophobic surfaces), for liquid impermeability. Various liquid impermeable, vapor permeable films and other materials are known and used in fields including personal hygiene, garment and wound dressing applications. A liquid impermeable but vapor permeable barrier layer may be preferred in some circumstances for purposes of venting water vapor to improve wearer comfort and/or help avoid overhydration of the wearer's skin. A monolithic thermoplastic elastomeric (TPE) film material may be desired for the barrier layer, for having stretch/elasticity characteristics and breathability.

It may also be desired in some examples that the material selected for the barrier layer be able to withstand application of heat, where used to bond the barrier layer to an overlying fabric layer, such that the material will not develop holes therethrough during the bonding process (which can compromise liquid impermeability). In some examples, a suitable barrier layer material for the above-mentioned purposes may include polyethylene terephthalate (PET), or thermoplastic polyester elastomer (TPEE). The material may be obtained in the form of a monolithic film and may be applied and bonded to a fabric layer via application of heat.

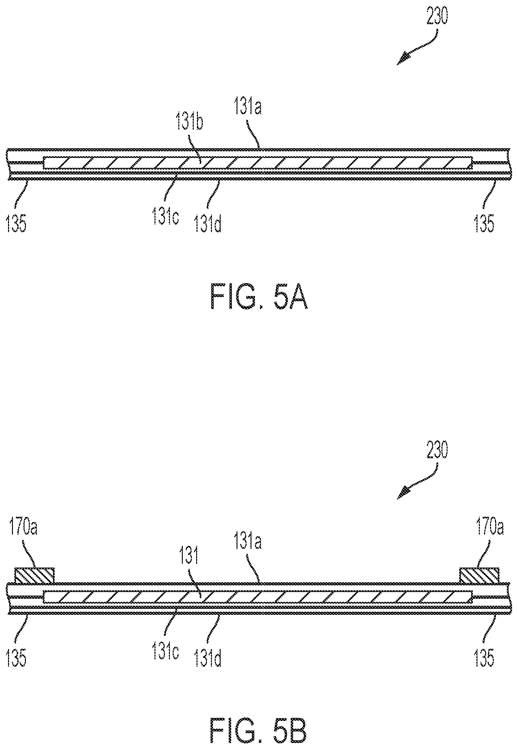

Referring to FIGS. 2B, 3B, 3D, and 5B, for purposes of reducing chances that urine or menses may escape to locations beyond overlying absorbent portions of the crotch portion 130 of the pant during wear, it may be desired to include one or more sections of containment barrier 170a, 170b, 170c. A containment barrier may be formed of any material suitable for creating a defined hydrophobic zone that resists wetting and flow of urine or menses thereover, or even a barrier or wall structure extending from the wearer-facing surfaces of the pant toward the wearer's skin and defined by a greater z-direction Caliper relative the central z-direction Caliper of the crotch portion 130 (i.e., at the intersection 250 of the longitudinal 200 and lateral 300 axes of the pant). As suggested in the figures, a pair of side containment barriers 170a may be located along the inside leg edges 140 of a brief pant (see, e.g., FIGS. 2B and 5B), and may be configured to follow the curvature of the leg edges 140. In other examples (not shown), side containment barriers may have other configurations, including but not limited to following straight lines or paths, or curvatures other than that of the leg openings.

Front 170b and/or rear 170c containment barriers may be located, respectively, proximate front and/or rear extents 401, 402 of the crotch portion 130 (see, e.g., FIGS. 2B, 3B, and 3D.) In some examples as suggested in FIG. 2B, a system of containment barriers may substantially or entirely circumscribe the intersection 250 of the longitudinal 200 and lateral 300 axes of the pant, and may substantially or entirely circumscribe the crotch portion 130. In some examples, a continuous containment barrier having effectively joined portions 170a, 170b, 170c may entirely circumscribe the intersection 250 of the axes, and may follow a path defining a circular shape, oval shape, stadium shape, rounded rectangle shape, peanut shape, hourglass shape or any other desired shape, substantially or entirely circumscribing the intersection 250. For purposes of minimizing chances of leakage, it may be desired that a continuous containment barrier entirely circumscribe the intersection 250, forming a liquid impermeable perimeter seal, as shown in FIG. 2B.

A containment barrier may be formed substantially of a hydrophobic material, for purposes of resisting wetting and flow of urine or menses thereover and/or therearound. Suitable hydrophobic materials include: a bead of hydrophobic polymeric material (e.g., a bead of a hydrophobic silicone-based compound), which may be applied in fluid form to the wearer-facing surface of the pant and then solidified (flocking may also be applied to the bead), a strip of hydrophobic film, hydrophobic closed-cell polymeric foam, or hydrophobic fabric, which may be affixed to the wearer-facing surface of the pant (e.g., by a flexible, extensible, hydrophobic adhesive), or a strip of hydrophobic film, hydrophobic closed-cell polymeric foam, or hydrophobic fabric, which may be affixed to the wearer-facing surface of the pant via thermal compression bonding. In other examples, a strip of hydrophobic tape (not shown) may be affixed via adhesive along the inside leg opening edges 140, and wrapped about the edges and around the outer fabric layer 131d, thereby sealing the edges of the layers at the leg edges and providing wearer-facing strip along the leg openings that hinders urine or menses flow thereover. The hydrophobic tape affixed along the inside leg opening edges and any material used to form the containment barrier may be selected to have elongation or elastic elongation attributes so as not to substantially reduce elastic elongation capabilities of the assembled layers present in the crotch portion 130.

Referring to FIGS. 2A and 2B by way of example, for purposes herein, crotch portion 130 is the portion of the pant, at a minimum, lying between crotch portion minimum front extent 401 and crotch portion minimum rear extent 402, longitudinally centered about crotch portion lateral axis 301 (which is drawn along the smallest width dimension CW measured between the crotch leg opening edges 140), plus 10 percent of the overall length L of the pant to the front and the rear of the crotch portion lateral axis 301. As suggested by FIGS. 2A and 2B, it may be desired that the crotch portion lateral axis 301 be disposed forward of the lateral axis 300 (which equally divides overall length L), rather than be co-located with lateral axis 300, for purposes of better fit about an adult female wearer's legs and lower torso. Thus, the respective boundaries between crotch portion 130 and front and rear waist portions 100, 120 for purposes herein are independent of the location(s) of any seams such as seams 134, 138 that may be present to join material(s) included in the crotch gusset 230 and material(s) included in the front and rear waist portions 100, 120.

Material(s) forming one or both of forward and rearward portions 132, 136 of crotch portion 130 may be continuous with material(s) forming front and rear waist portions 100, 120, or alternatively, one or both of forward and rearward portions 132, 136 of crotch portion 130 and/or crotch gusset 230 may be substantially formed of one or more sections or layers of material that are distinct from material(s) substantially forming one or both of front and rear waist portions 100, 120, and crotch gusset 230 may be joined to front and rear waist portions 100, 120 at one or both of forward seam 134 and rearward seam 138. In illustrative but non-limiting examples reflected in FIGS. 3A and 3B, front waist portion 100, an outward-facing layer of crotch portion 130, and rear waist portion 120, may be formed partially or entirely of a first single, continuous section of material. As shown in FIGs. 3B-3E, one or more additional layers of material (wearer-facing, liquid permeable top layer 131a and durable absorbent layer 131b) may be added to crotch portion 130 on the wearer-facing side as shown, and be affixed to the first section of material via, e.g., stitching/sewing, adhesive bonding, thermal bonding (fusing or welding) or other suitable attachment/joining mechanism (hereinafter, attachment mechanism) at forward and rearward seams 134, 138. Optionally these layers may be offset from the forward and rearward seams to reduce bulk and stacking of seamed layers directly overlaying each other, which may enhance comfort, as described in US Provisional Patent Application No. 63/315188, incorporated by reference in its entirety herein.

In other illustrative but non-limiting examples, referring to FIGS. 3C and 3D, the sections of materials respectively forming front waist portion 100, rear waist portion 120 and crotch gusset 230 may be separate and distinct, and joined via any suitable attachment mechanism at forward and rearward seams 134, 138. A configuration approach schematically depicted by way of non-limiting examples in FIGS. 3C and 3D may be preferred because these approaches may provide the designer with greater flexibility in selection of the respective materials for the waist and crotch portions with respect to appearance, feel, weight, breathability, elongation, stretch characteristics and cost. The elongation and stretch characteristics described below would be applicable to the one, or more layers of material in combination, present in crotch portion 130 and/or crotch gusset 230.

For purposes that may include odor control, in some examples any, some or all of the materials present in the crotch gusset 230, including but not limited to the wearer-facing, liquid permeable top layer 131a and the durable absorbent layer 131b of the durable absorbent panel may be formed of component material(s) that include a metal, metal alloy or metallic compound having antimicrobial properties. Examples may include copper, silver, zinc and aluminum, and alloys and/or compounds thereof. The incorporation or "infusing" of such materials into threads or yarns that may knitted is known in the art. Other suitable odor control agents may also be incorporated.

When the durable absorbent brief pant is fully assembled the front and rear waist portions 100, 120 are joined together at left and right side hip portions 160, resulting in the formation of a waist opening, including waist opening edges (102 and 122), and left and right leg openings, including left and right leg opening edges 145 (See FIG. 1). In some examples, the front and rear waist portions 100, 120 may be formed of a single, continuous section of material without side seams ("seamless" construction). In other examples, the front and rear waist portions 100, 120 may be formed of distinct sections or separated portions of material and joined at hip side seams extending approximately vertically or longitudinally along the hip portions 160.

Any or all of front waist portion 100, rear waist portion 120 and crotch portion 130 may be formed of a knitted or a woven material. A knitted material may be a stretch knit material that includes elastic strands or threads that impart to, or enhance, elastic extensibility and contractibility of the material. A variety of suitable knitted and stretch knit materials having varying and selectable elongation and tensile modulus properties are known in the arts of garment manufacturing and are commercially available. Suitable stretch knit materials are currently available from Schoeller Textil AG, Sevelen, Switzerland. Knitted and woven materials are described in greater detail below. Suitable materials may be selected for their elongation and tensile modulus properties, and also for their weight, breathability, moisture wicking properties, tactile feel against the skin and cost. In some examples the material may be knitted from non-elastic and elastic fibers, threads or yarns, where the elastic components are formed of or include an elastomeric material such as elastane (for example, spandex or LYCRA, available from The Lycra Company, Wilmington, Delaware).

The durable absorbent brief pant may be further configured with a waist band structure (not specifically shown) at the waist edges, and leg band structures (not specifically shown) at the leg opening edges. Waist band and leg band structures may be formed of folded-over portions of the materials forming the front waist portion, rear waist portion and/or crotch portion. In some examples a plurality of strands or one or more strips of elastomeric material may be captured and contained within the folded-over portions, to supplement elasticity and contractile forces within the material itself, improve the ability of the durable absorbent pant to stay in place on the wearer's body during wear, and to contain urine or menses. In some examples, a separate and distinct waist band assembly and/or leg band assemblies may be provided and affixed via suitable attachment mechanism, to the front waist, rear waist and crotch portions proximate to, or in a position to define, the waist opening edge and leg opening edges. Elastic band assemblies suitable for forming separate waist bands and leg bands for underwear such as a durable absorbent brief pant are known in the arts of garment manufacturing and are commercially available. Elastic waist and leg band structures may be desired to better hold the pant in place on the wearer's body, and elastic leg band structures may be desired further to provide supplemental tensile force about the leg openings that helps hold a removable absorbent insert against the wearer's body in the crotch region of the body.

A durable absorbent pant having any desired combination of the features described herein may be effectively used in combination with one or more removable absorbent inserts. FIG. 3E depicts an example of a durable absorbent brief pant adapted or configured to accommodate one or more removable absorbent inserts 150. The durable absorbent brief pant of FIG. 3E comprises a crotch portion 130 comprising a gusset 230, where the gusset 230 comprises a durable absorbent panel comprising a wearer-facing, liquid permeable top layer 131a, a durable absorbent layer 131b below the wearer-facing liquid permeable top layer, and an outward-facing, liquid impermeable barrier layer 131c subjacent to the durable absorbent layer. FIG. 3E may be formed of and/or include materials, similar to the example depicted in FIG. 3B described above, but with the enveloping structure 151 and lacking one or more sections of containment barrier 170b, 170c. As shown in FIG. 3E, the wearer-facing, liquid permeable top layer 131a and the durable absorbent layer 131b form an enveloping structure 151 having a closed end and an open end adapted to receive one or more removable absorbent inserts 150 in a position below the wearer-facing, liquid permeable top layer 131a of the durable absorbent panel. The open end of the enveloping structure 151 may comprise a slit 133 in the wearer-facing, liquid permeable top layer of the durable absorbent panel. The slit 133 may optionally be reinforced (not shown). There are various means to reinforce the slit, including with stitching, folding of materials, folding a cut edge underneath and sealing or stitching in place, and/or application of tapes or vinyl appliques (on the wearer-facing side, the outward-facing side, or both). The open end may also be formed by folding back the liquid permeable top layer 13 1a or using multiple, separate pieces of liquid permeable top layer 131a material, similar to a pillowcase opening.

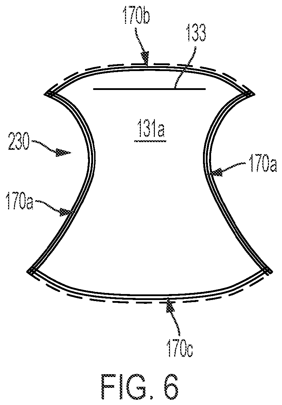

Referring to FIG. 6, the wearer-facing, liquid permeable top layer 131a of the pant may be bonded to the subjacent durable absorbent layer 13 1b of the pant, outboard of the enveloping structure 151. In some examples, bonds between the layers may be formed by discrete deposits of adhesive between the layers, outboard of the enveloping structure 151, to adhere the layers together at the locations of the deposits. In other examples, bonds between the layers may be formed, outboard of the enveloping structure 151, by thermal compression bonding, which may be more durable through a plurality of launderings. For purposes of enabling thermal compression bonding, the wearer-facing layer and the subjacent absorbent layer may each include polymer components of respective fusible compositions (such as similar polyester-based compositions) that facilitate formation of robust bonds between the layers upon localized application of heat and pressure at the bond sites. (Herein, "fusible compositions," with respect to two respective polymers present in two respective layers, means that the two polymers are miscible, capable of melting and mixing at a temperature of 250 °C or lower, to form a single thermodynamic phase.) If the pant comprises one or more section of containment barrier, particularly a continuous containment barrier that entirely circumscribes the intersection 250 to form a liquid impermeable perimeter seal (as shown in FIG. 2B), the enveloping structure 151 is preferably inboard of the one or more sections of containment barrier (said differently, the one or more sections of containment barrier are outboard of the enveloping structure 151).

The one or more removable absorbent inserts may each have a bending stiffness in the machine direction (MD)/longitudinal of about 10 N/m$^2$ to about 100 N/m$^2$, preferably about 10 N/m$^2$ to about 80 N/m$^2$, more preferably about 20 N/m$^2$ to about 50 N/m$^2$. The one or more removable absorbent inserts may each have a bending stiffness in the cross direction (CD)/lateral of about 10 N/m$^2$ to about 100 N/m$^2$, preferably about 10 N/m$^2$ to about 80 N/m$^2$, more preferably about 20 N/m$^2$ to about 50 N/m$^2$. Without wishing to be bound by theory, it is believed that there are preferred ranges of bending stiffness in the machine direction (MD) and the cross direction (CD) and, within these preferred ranges, the comfort and ease of insertion of the removable absorbent insert into the pant are optimized. Bending stiffness can be managed via the selection of component materials, e.g., caliper of component materials, and also via single- or multi-directional activation of component materials using processes such as embossing, ring rolling, bending, scoring, and/or folding.

The one or more removable absorbent inserts may each have a capacity of about 2 g to about 100 g, preferably about 5 g to about 80 g, more preferably about 10 g to about 50 g, even more preferably about 15 g to about 40 g. The one or more removable absorbent inserts may each have a caliper of about 0.5 mm to about 7 mm, preferably about 0.5 mm to about 5 mm, more preferably about 1.0 mm to about 3.0 mm. The one or more removable absorbent inserts may be durable or disposable, for example, flushable. The dimensions of the inserts generally match the dimensions of the crotch portion and/or the crotch gusset of the durable absorbent pant (for example, 19 cm by about 5.7 cm).

As suggested in the figure, the enveloping structure 151 may be open along a location such as along a front location of crotch portion 130, so as to provide a pocket that permits insertion and removal of the removable absorbent insert(s) 150. The removable absorbent insert(s) 150 may be durable or washable, but is preferably configured for single use and then disposal, being formed of and including materials similar to those typically included in a disposable feminine hygiene pad or incontinence pad. In some embodiments, each of the one or more removable absorbent inserts comprises a pull tab, as shown in FIG. 3E, to facilitate removal of the one or more removable absorbent inserts from the enveloping structure 151. The pull tab may be resistant to wetting by urine, menses, or other fluids. Preferably, the pull tab is hydrophobic. The pull tab or any portion of the removable insert may have a color that is similar to or matched to any portion of the durable underwear to reduce visual contrast. The portion that is a different color may also serve as an indicator for how far to insert the removable insert through the slit.

The typical insert is sized and shaped so as to be held within the enveloping structure of the durable absorbent pant, reasonably comfortably, against the user/wearer's body in the crotch region. Removable absorbent inserts typically include an insert (outward-facing) liquid impermeable backsheet, an insert absorbent core disposed above the insert liquid impermeable backsheet, and an optional insert liquid permeable topsheet disposed above the insert absorbent core (not shown). The insert absorbent core may include absorbent materials or accumulations and/or combinations thereof that volumetrically expand or swell as they absorb liquid, for example, accumulations of particles of absorbent gelling material (also known as superabsorbent polymer), batts of cellulose fibers, collapsed, open-cell absorbent foams (such as collapsed open-cell foams formed from High Internal Phase Emulsions (HIPEs)), or a combination thereof. It will be appreciated that a brief pant having features as described herein is particularly suited to accommodating such expansion or swelling as it aggregates to swell the insert as a whole, while maintaining an improved level of contact between the insert and the user/wearer's body.

In use, the one or more removable absorbent inserts, each insert comprising an insert liquid impermeable backsheet and an insert absorbent core disposed above the insert liquid impermeable backsheet, is inserted into the durable absorbent pant such that the insert absorbent core is positioned subjacent to the wearer-facing, liquid permeable top layer of the durable absorbent panel. The one or more removable absorbent inserts may optionally further comprise an insert liquid permeable topsheet disposed above the insert absorbent core; in use, the insert is inserted into the durable absorbent pant such that the insert liquid permeable topsheet is positioned subjacent to the wearer-facing, liquid permeable top layer of the durable absorbent panel. Importantly, unlike most conventional pads and liners, in preferred embodiments, the outward-facing surface of the liquid impermeable backsheet of each of the one or more removable absorbent inserts disclosed herein is substantially free of adhesive, to facilitate removal of the one or more removable absorbent inserts from the enveloping structure.

The one or more removable absorbent inserts may further comprise additional layers, such as a distribution layer or a dewatering layer. Distribution layers may be present in the insert to help move a soil and utilize more of the absorbent capacity of the insert. The dewatering layer may also be designed to efficiently pull fluid from the liquid permeable top layer of the durable absorbent pant.

Preferably, a kinetic coefficient of friction between the durable absorbent layer of the durable absorbent panel and the liquid impermeable backsheet of the removable absorbent insert superjacent the durable absorbent layer of the durable absorbent panel is about 0.1 to about 0.9, preferably about 0.3 to about 0.7. Preferably, a kinetic coefficient of friction between the wearer-facing, liquid permeable top layer of the durable absorbent panel and the liquid permeable topsheet of the removable absorbent insert subjacent to the wearer-facing, liquid permeable top layer of the durable absorbent panel is about 0.1 to about 0.9, preferably about 0.4 to about 0.8. The one or more removable absorbent inserts may each comprise a removable stiffening layer to facilitate insertion of the one or more removable absorbent inserts into the enveloping structure.

As noted above, in some embodiments, the durable absorbent pant disclosed herein may be combined or used with more than one removable absorbent insert, such as a bundle or a stack of two, three, four, or more removable inserts, as described in EP 0861641 A2, incorporated by reference in its entirety herein. The durable absorbent pant disclosed herein may be used with a stack of two to six removable absorbent inserts, preferably a stack of two to four removable absorbent inserts, more preferably a stack of three removable inserts, where the first removable absorbent insert is positioned subjacent to the wearer-facing, liquid permeable top layer of the durable absorbent panel, the second removable absorbent insert is positioned subjacent to the first removable absorbent insert, and the third removable absorbent insert is positioned subjacent to the second removable absorbent insert.

In use, when the top-most removable insert is saturated with fluid, a user may remove it from the stack of inserts (and optionally dispose of it), thereby exposing the fresh insert below it. When the top-most removable insert is removed, the fresh insert below it may dewater the wearer-facing, liquid permeable top layer of the durable absorbent panel of the pant, thereby providing the user with a fresh and dry feeling pant. The individual inserts in the stack of inserts may be non-joined or joined in any manner that allows each insert to be readily removed from the stack, e.g., peeled off, one after another. For example, the edges of the inserts may be joined together using a peelable adhesive, a peelable embossing, or a combination thereof. Importantly, as discussed above, the outward-facing surface of the liquid impermeable backsheet of each of the one or more removable absorbent inserts is substantially free of adhesive, embossing, or other joining mechanisms. In other words, any adhesive, embossing, or other joining mechanism is limited to the edges of the inserts, to facilitate easy removal of each insert from the enveloping structure.

The durable absorbent pant may have a number of additional features that improve containment performance, when the pant includes or is used with a removable absorbent insert.

Crotch Portion / Crotch Gusset

Width

It may be desired that the durable absorbent brief pant have sufficient width in the crotch portion, particularly across a range of pant sizes designed to fit different body sizes, shapes, and types of users. Thus, regardless of the size of durable absorbent pant, the removable absorbent insert designed for insertion into the pant has a standardized width or standardized width and length, because the crotch portion of the pant, regardless of pant size, is of sufficient width to accommodate the insert. Accordingly, it may be desired that the crotch portion 130 have a relaxed width CW, measured from right leg edge to left leg edge at the narrowest part of the crotch portion, of at least 5.5 cm, or at least 6.0 cm, and less than about 11 cm, preferably less than about 10 cm, and more preferably less than 9 cm.

Caliper

For purposes of minimized bulk, it may be desired that the combination of layered materials present in the crotch portion 130 and/or crotch gusset 230 be selected and configured so has to have a central z-direction Caliper not exceeding about 5 mm, more preferably not exceeding about 4 mm, and even more preferably not exceeding about 3.5 mm. (Herein, the "central z-direction Caliper" or "central Caliper" is measured at a location in the crotch portion 130 corresponding with the intersection 250 of the longitudinal 200 and lateral 300 axes of the pant, when opened and laid out flat on a horizontal planar surface.) Based on disclosure herein and teachings available in the art concerning textiles, persons of ordinary skill in the art will be equipped to select a combination of suitable materials to create a structure in the crotch portion 130 such as a crotch gusset 230 having such Caliper, along with other features and attributes described herein.

Absorption Capacity

In order for a durable absorbent pant to provide suitable absorbency and protection against leakage of unintended discharges of urine or menses over a reasonable duration of wear, while balancing concerns for limiting caliper/bulkiness of the crotch portion and while limiting the relative planar size/surface area of an absorbent portion, it may be desired that a combination of material forming the structure within the crotch portion 130 of the pant have an Area Absorption Capacity from 0.1 ml/cm$^2$ to 1.0 ml/cm$^2$, and a Volume Absorption Capacity from 0.4 ml/cm$^3$ to 0.8 ml/cm$^3$, measured according to the Absorption Capacity Measurement method set forth below. Based on disclosure herein and teachings available in the art concerning textiles, persons of ordinary skill in the art will be equipped to select a combination of suitable materials to create a structure in the crotch portion 130 having such absorption capacity. It is believed, further, based on recent availability of fabric materials including yarns that include superabsorbent polymers (e.g., fabrics including "SAF" superabsorbent fiber, as available from Technical Absorbents Limited, Grimsby, United Kingdom), that a crotch portion may be configured to have a Volume Absorption Capacity greater than 0.8 ml/cm$^3$, e.g., up to 0.9 ml/cm$^3$, 1.0 ml/cm$^3$ , 1.1 ml/cm$^3$, or even up to 1.2 ml/cm$^3$. Without intending to be found by theory, it is believed that materials such as these can provide Volume Absorption Capacity at such levels by swelling beyond their dry volumes, with absorbed liquid.

Stretch Attributes

It may be desired that the crotch portion 130 exhibit a sufficient degree of longitudinal and lateral elongation to accommodate an increase in bulk of an absorbent insert resulting from absorption and swelling, and prevent swelling of the insert from creating gaps between, e.g., leg bands and the wearer's skin. At the same time, it may be desired that longitudinal elongation be more limited than lateral elongation, to help maintain a pad within the crotch portion in position against the wearer's body. Accordingly, it may be desired that the crotch portion exhibit lateral Elongation of 50 percent to 200 percent, and a longitudinal Elongation of 5 percent to 100 percent. In some examples, it may be desired that materials be selected to form the crotch portion such that they result in a ratio of lateral Elongation to longitudinal Elongation be 3:1 or greater.

In combination with longitudinal and lateral elongation capability, it may be desired that the crotch portion have Tensile Modulus properties that are selected to strike a balance between suitably allowing the crotch portion to expand in width and length. Accordingly, it may be desired that the crotch portion be formed of material(s) that impart to it a longitudinal Tensile Modulus of 10 gf/mm to 150 gf/mm, and a lateral Tensile Modulus of 2 gf/mm to 75 gf/mm, more preferably 2 gf/mm to 40 gf/mm, and even more preferably 2 gf/mm to 20 gf/mm. In some examples, it may be desired that materials be selected to form the crotch portion such that they result in a ratio of longitudinal Tensile Modulus to lateral Tensile Modulus of 3:1 or greater.

It will be appreciated from the description above that, generally, the stretch characteristics desired for the material(s) of the crotch portion, are, generally, relatively higher Elongation and relatively lower Tensile Modulus in the lateral direction, in combination with relatively lower Elongation and relatively higher Tensile Modulus in the longitudinal direction.

In the ranges of values for Elongation and Tensile Modulus set forth above, it is contemplated that any combination of narrower ranges (sub-ranges) within the broader ranges set forth may be selected under particular circumstances, as may be desired according to, for example, pant size and style (e.g., bikini, hip hugger, control brief, etc.), elongation and modulus features of material(s) used to form waist portions, etc. By way of non-limiting example, selected sub-ranges for crotch lateral Elongation may be 50 percent to 100 percent, or 100 percent to 200 percent, or 75 percent to 150 percent, etc., all being within the broader range set forth above, and all such permutations of sub-ranges being within contemplation of the present disclosure.

For purposes herein, Elongation and Tensile Modulus are measured using the methods set forth below.

In combination with either or both the above features of Elongation and Tensile Modulus and in some examples in combination particularly with the above-described Elongation, it may also be desired that material(s) used to form the crotch portion not impart the crotch portion with an excessive Poisson Contraction Effect upon lateral stretching/extension. For purposes herein, the Poisson Contraction Effect quantifies and expresses the extent to which a material will contract along a first direction in a plane, when elongated/strained along a second direction in the plane perpendicular to the first direction. When the crotch portion is strained/elongated in a lateral direction, excessive Poisson Contraction Effect in the longitudinal direction can work against the purpose of allowing expansion of the crotch portion to accommodate an increase in bulk from an insert. Accordingly, it may be desired that the material(s) of the crotch portion be selected such that as assembled they will exhibit Poisson Contraction Effect, resulting from to lateral stretch/extension, no greater than 0.4, more preferably no greater than 0.2, and even more preferably no greater than 0.15. For purposes herein Poisson Contraction Effect measured according to the Poisson Contraction Effect method set forth below.

Many knitted fabrics exhibit anisotropic planar tensile elongation properties, having a maximum elongation capability along a first direction and a minimum elongation capability along a second direction substantially perpendicular to the first direction. Woven fabrics of some types also exhibit anisotropic planar tensile elongation properties, having a maximum elongation capability along a first direction and one or two lesser and/or minimum elongation capabilities along the two directions that are substantially 45 degrees from the first direction. For purposes of providing herein-described elongation capabilities to the crotch portion 130, it may be desired the material(s) selected to form one or all layers of the crotch portion be oriented within the crotch portion such that their directions of maximum elongation capability lie substantially along the lateral direction, relative the brief pant. It may be further desired that knitted fabric(s) be used to form one, more or all of the wearer-facing, outward-facing and any intermediate layers of the crotch portion because knitted fabric(s) of selected knit types may be identified that exhibit the desired limited Poisson Contract Effect as discussed above.

In some examples, the material selected to form or be included in crotch portion 130 may be a rib knit fabric. The ribs of the knit may be oriented so as to be substantially aligned with the longitudinal direction. In this orientation, the particular configuration of rib knit fabric permits it to elongate substantially in the lateral direction with relatively low accompanying longitudinal contraction. Thus, a rib knit fabric with ribs oriented substantially longitudinally may be desired for purposes of providing the benefits of limited Poisson Contraction Effect as discussed above.

Where the crotch portion is formed of or includes only a single layer of material, the material forming the single layer may be selected so as to have the stretch characteristics described above. Where the crotch portion is formed of a plurality of layers as suggested in, by way of example, FIGs. 3A-3E, the materials forming the plurality of layers may be selected so as to have, in combination, the stretch characteristics described above. Where the crotch portion is configured to contain a durable absorbent layer 131b, as shown in FIGs. 3A-3E, material(s) forming the outer fabric layer 131d may be selected so as to have the stretch characteristics described above. Alternatively, all materials forming the wearer-facing, liquid permeable top layer 131a and the optional outer fabric layer 131d may be selected so as to have the stretch characteristics described above, in combination.

In combination with the features described above applicable to the materials of the crotch portion 130, materials forming or included by the waist portions 100, 120 may be selected so as to have particular stretch characteristics that cooperate with the materials of the crotch portion to have the desired expandable but snug insert holding properties of the materials of the crotch portion. Accordingly, it may be desired that one or both of the front waist portion 100 and rear waist portion 120 be formed of or include knit material(s) that in combination, for the waist portion: a maximum longitudinal elongation of 200% and a longitudinal tensile modulus of 5 gf/mm to 25 gf/mm, more preferably 10 gf/mm to 20 gf/mm, and even more preferably 13 gf/mm to 17 gf/mm; and a maximum lateral elongation of 200% and a lateral tensile modulus of 5 gf/mm to 25 gf/mm, more preferably 10 gf/mm to 20 gf/mm, and even more preferably 13 gf/mm to 17 gf/mm.

Arrays of Products

It is contemplated that a brief pant product within contemplation of the description above may be offered for sale as one of two or more products in an array that includes one or more removable absorbent insert products. In some examples, the respective products of the array can be accompanied by information matching or coupling them not only by associated trademark, but also by, e.g., advertised size, advertised use (e.g., "moderate" or "light" incontinence needs, "daytime" or "nighttime" use, etc.), pant design (e.g., bikini, hip hugger, control brief, etc.), complementary or compatible lengths, crotch portion widths or other dimensions, or other features making them particularly adapted, complementary or compatible for use with one another.

More specifically, it is contemplated to provide an array of packages comprising two or more different sizes of durable absorbent pants adapted to accommodate one or more removable absorbent inserts, the array comprising: a. a first package and a second package, where the first package comprises a first durable absorbent pant being a first size and the second package comprises a second durable absorbent pant being a second size, where the second size is different than the first size, where each of the first and second durable absorbent pants comprises: i. a front waist portion with a front waist edge and left and right front leg opening edges; ii. a rear waist portion with a rear waist edge and left and right rear leg opening edges; iii. a crotch portion comprising a gusset, the crotch portion having a forward portion meeting the front waist portion and a rearward portion meeting the rear waist portion; and iv. left and right hip side portions joining the front waist portion to the rear waist portion and thereby forming a waist opening with a waist opening edge comprising the front waist edge and the rear waist edge, and left and right leg openings; where each of the front waist portion, rear waist portion and gusset comprises a knitted or woven material; where the gusset comprises a durable absorbent panel comprising a wearer-facing, liquid permeable top layer, a durable absorbent layer subjacent to the wearer-facing liquid permeable top layer, a liquid impermeable barrier layer subjacent to the durable absorbent layer, the wearer-facing liquid permeable top layer and the durable absorbent layer forming an enveloping structure 151 having a closed end and an open end adapted to receive the one or more removable absorbent inserts in a position subjacent to the wearer-facing, liquid permeable top layer of the durable absorbent panel. The array of may further comprise a third package comprising at least a first removable absorbent insert and a second removable absorbent insert, where the length and/or width of the first removable absorbent insert is about the same as the length and/or width of the second removable absorbent insert, the first and the second removable absorbent inserts each being configured for insertion into either the first or the second durable absorbent pant. The first and second removable absorbent inserts have substantially the same features or the first removable absorbent insert may have one or more features that substantially differ from one or more features of the second removable absorbent insert. For example, the first removable absorbent insert may have a first capacity and the second removable absorbent insert may have a second capacity.

It is also contemplated to provide an array of packages comprising a durable absorbent pant adapted to accommodate one or more removable absorbent inserts, the array comprising: a. a first package, where the first package comprises a durable absorbent pant comprising: i. a front waist portion with a front waist edge and left and right front leg opening edges; ii. a rear waist portion with a rear waist edge and left and right rear leg opening edges; iii. a crotch portion comprising a gusset, the crotch portion having a forward portion meeting the front waist portion and a rearward portion meeting the rear waist portion; and iv. left and right hip side portions joining the front waist portion to the rear waist portion and thereby forming a waist opening with a waist opening edge comprising the front waist edge and the rear waist edge, and left and right leg openings; where each of the front waist portion, rear waist portion and gusset comprises a knitted or woven material; where the gusset comprises a durable absorbent panel comprising a wearer-facing, liquid permeable top layer, a durable absorbent layer subjacent to the wearer-facing liquid permeable top layer, a liquid impermeable barrier layer subjacent to the durable absorbent layer, the wearer-facing liquid permeable top layer and the durable absorbent layer forming an enveloping structure 151 having a closed end and an open end adapted to receive the one or more removable absorbent inserts in a position subjacent to the wearer-facing, liquid permeable top layer of the durable absorbent panel; b. a second package, where the second package comprises a first removable absorbent insert having a first capacity and configured for insertion into the durable absorbent pant; c. a third package, where the third package comprises a second removable absorbent insert having a second capacity and configured for insertion into the durable absorbent pant; where the length and/or width of the first removable absorbent insert is about the same as the length and/or width of the second removable absorbent insert.

Measurement Methods

General Sample Preparation

Each of the measurements below is to be conducted on 10 separate like samples (from 10 separate like samples of brief panties) and the average of the 10 separate like samples is considered to be the measurement for that specific sample set.

Samples are collected from the crotch portion (130) of the pant. Samples are cut from about the longitudinal and lateral center of the crotch portion. In testing for longitudinal properties, samples are cut in a rectangular shape, 6 cm long (dimension measured in the longitudinal direction) and by 2 cm wide (dimension measured in the lateral direction). In testing for lateral properties, samples are cut in a rectangular shape, 6 cm wide (dimension in the lateral direction) by 2 cm long (dimension measured in the longitudinal direction).

The sample should be cut with a sharp knife or suitably sharp cutting device designed to precisely cut the sample. Data collection is accomplished through a combination of manual measurements, machine elongation measurements and machine load measurements.

The testing is performed under ambient room conditions (temperatures from between 15°C to 35°C and relative humidity from between 35% to 75%). Samples are conditioned for at least two hours prior to testing under the same conditions.

All linear dimensions are measured manually by ruler within the ordinary x-y plane, using a ruler that is traceable to NIST or other standards organization.

Tensile Modulus

The Tensile Modulus of the sample is determined by stretching in the direction of the sample's longer dimension using a constant rate of extension tensile testing machine with computer interface, *e.g.*, Instron; MTS; Zwick; etc., using a load cell for which the loads measured are within 10% to 90% of the limit of the cell, and ensures accuracy of a 5N load to 0.1N. The instrument is equipped with a single line contact grips, wider than 3 cm (ensures wider than the short axis of the sample). Prior to testing, calibrate the equipment according to the instruments manufacturer's recommendations.

The grips of the tensile testing machine consist of air actuated grips designed to hold the sample. No slippage should be permitted between the sample and the grips. The distance between the lines of gripping force (distance from one grip to the next, along the axis of the machine's elongation) should be 3 cm as measured by a steel rule held beside the grips. This distance will be referred to from here on as the "starting gauge-length".

The sample is mounted in the grips with its long axis parallel to the direction of applied elongation, and the center line of the long axis of the sample centered in each grip. 1 cm of each end of the sample's long axis is inserted into each grip, leaving 4 cm of the long axis of the sample between the grips. The starting gauge-length of 3 cm ensures some slack in the sample at the start of the test.

After the sample is mounted, the machine's load channel is set to zero (this eliminates the weight of the sample in the calculations). The grips are slowly moved apart at 5.08 cm/min (2.0 in/min) until a load of 5 gf (grams-force) is attained. The separation between the grips at this position is recorded as $L_0$.

$$(L_0 = \text{starting gauge-length} + \text{additional machine extension to reach 5 gf}).$$

After the 5 gf load is attained, extend the sample at a rate of 50.8 cm/min (20 in/min) with a data acquisition rate of 50 Hz. Extend until either a stress of 15 gf/mm is reached, or the sample breaks.

For stress calculation, the thickness of the material is neglected, hence the stress values are recorded in units of gf/mm. Stress is calculated by dividing the load in the sample, as recorded by the load cell, by the sample width (short axis as measured in units of mm).

Sample strain is calculated by $\Delta L/L_0$. $\Delta L$ is any additional extension between the grips after $L_0$ is reached and is recorded along with load at a rate of 50 Hz. Sample strain is expressed numerically (not as a percentage), thus a strain of 100% is 1.0 for the purposes of these calculations.

Record the sample strains at sample stresses of 10 gf/mm and at 20 gf/mm.

Tensile Modulus is the linear slope between 10 gf/mm and 20 gf/mm, and is calculated as:

Tensile Modulus = [20 gf/mm - 10 gf/mm] / [sample strain at 20 gf/mm - sample strain at 10 gf/mm]

Repeat for 10 samples.

Elongation

The Elongation is measured during the Tensile Modulus test. The Elongation is the sample strain at a sample stress of 20 gf/mm. Elongation is expressed as a percent strain, e.g., a value of 1.0 strain from the Tensile Modulus method is expressed as 100% strain for Elongation.

Repeat and record the results for 10 samples. Calculate and record the average of the results. The average will be the Elongation value for the subject pant design.

Poisson Contraction Effect

The Poisson Contraction Effect is measured by stretching the sample in the direction of the longer dimension of the sample using a constant rate of extension tensile testing machine with computer interface, *e.g.*, Instron; MTS; Zwick; etc., using a load cell for which the loads measured are within 10% to 90% of the limit of the cell, and ensures accuracy of a 5N load to 0.1N. The instrument is equipped with a single line contact grips, wider than 3 cm (ensures wider than the short axis of the sample). Prior to testing, calibrate the equipment according to the instruments manufacturer's recommendations.

The grips of the tensile testing machine consist of air actuated grips designed to hold the sample. No slippage should be permitted between the sample and the grips. The distance between the lines of gripping force (distance from one grip to the next, along the axis of the machine's elongation) should be 3 cm as measured by a steel rule held beside the grips. This distance will be referred to from here on as the "starting gauge-length".

The sample is mounted in the grips with its long axis parallel to the direction of applied elongation, and the center line of the long axis of the sample centered in each grip. 1 cm of each end of the sample's long axis is inserted into each grip, leaving 4 cm of the long axis of the sample between the grips. The starting gauge-length of 3 cm ensures some slack in the sample at the start of the test.

After the sample is mounted, the machine's load channel is set to zero (this eliminates the weight of the sample in the calculations). The grips are slowly moved apart at 5.08 cm/min (2.0 in/min) until a load of 5 gf (grams-force). The separation between the grips at this position is recorded as $L_0$.

$$(L_0 = \text{starting gauge-length} + \text{additional machine extension to reach 5 gf}).$$

After the 5 gf load is attained, extend the sample at a rate of 50.8 cm/min (20 in/min) with a data acquisition rate of 50 Hz. Extend until a stress of 7 gf/mm is reached. The machine's extension is stopped at the point, and the sample is held at this stress and accompanying strain.

For stress calculation, the thickness of the material is neglected, hence the stress values are recorded in units of gf/mm. Stress is calculated by dividing the load in the sample, as recorded by the load cell, by the sample width (short axis).

Sample strain is calculated by $\Delta L/L_0$. $\Delta L$ is any additional extension between the grips after $L_0$ is reached and is continuously recorded along with load at a rate of 50 Hz.

The narrowest width of the sample perpendicular to its long axis is measured to the nearest 0.5 mm using a steel rule ($W_n$). The narrowest width is typically at the center of the sample along its longer dimension.

The Poisson Contraction Effect (PCE) is calculated by:

$$PCE = [(W_0 - W_n)/W_0] \, / \, [\Delta L/L_0]$$

Where:

$W_0$ = sample lateral dimension prior to stretching

$W_n$ = the narrowest dimension of the sample perpendicular to the stretch direction after stretching to a stress of 7 gf/mm

$L_0$ = starting gauge-length + additional machine extension to reach 5 gf

$\Delta L$ = additional extension between the grips after $L_0$ is reached at a sample stress of 7gf/mm

Repeat and record the results for 10 samples. Calculate and record the average of the results. The average will be the Poisson Contraction Effect value for the subject pant design.

Liquid Impermeability

When there is a question concerning whether a particular pant has a gusset with a liquid impermeable barrier layer, this Liquid Impermeability test method may be used to measure a quantity of test liquid that will pass through a sample and enable determination whether there is a "liquid impermeable barrier layer" present in the gusset, according to the definition set forth in the description above.

The Liquid Impermeability test measures the quantity of liquid transferred through to the outward-facing side of a test specimen obtained from a pant after it is dosed with a prescribed volume of test liquid in order to simulate a liquid insult during actual use/wear of the pant.

All testing is performed in a room controlled at 23°C $\pm$ 3C° and 50% $\pm$ 2% relative humidity.

Prior to performing the measurement of this method, the examples of the pants of interest (prior to removal of samples therefrom) are washed in order to mimic in-use conditions and to follow the recommended "prior to use" instructions that accompany these types of pants (e.g. wash before use). The examples are placed into a mesh lingerie bag, and then placed into a high efficiency, front-loading washing machine (any convenient source) along with a single small/light load dosage of TIDE brand laundry detergent ("Original" designation; "HE" or other high efficiency washing machine designation; without additives such as FEBREZE, ODOR DEFENSE, OXI additives, bleach or bleaching additives or fabric softening additives) (product of The Procter & Gamble Company, Cincinnati, Ohio), or equivalent. The washer is set to delicate cycle using cold water. After the wash cycle, the examples are removed from the mesh bag and placed flat on a drying rack to air dry for about 12 hours. After air-drying, the examples are placed into a clothes dryer (any convenient source) set on the delicate cycle with very low heat for about 5 minutes or until dry to the touch.

Test samples are prepared as follows. The pre-washed and dried example pants are equilibrated in a room controlled at 23°C $\pm$ 3C° and 50% $\pm$ 2% relative humidity for about 2 hours. Test samples containing the entire gusset are removed from the examples as described in the General Sample Preparation section herein. Using scissors, the outermost fabric layer on the outward-facing side of the gusset is cut out along the entire gusset shape, inboard of the seams, using care so as not to cut into any of the edge seams present. Mark the dose location at the intersection of the midpoint of the longitudinal axis of the sample and a lateral axis positioned at the narrowest portion of the specimen.

For each test sample, a single layer of filter paper is cut to 10 cm by 2.54 cm. A suitable filter paper is Ahlstrom Grade 989 (available from Ahlstrom-Munksjo North America LLC, Alpharetta, GA), or equivalent. The test liquid is deionized water at room temperature (23°C + 3C°).

Record the mass of one layer of pre-cut filter paper and record as Dry Mass$_{fp}$ to the nearest 0.0001 grams. Place the pre-weighed filter paper onto a flat horizontal work surface. Position the test specimen centered over the filter paper with the garment facing side of the specimen facing the paper. Using a volumetric pipette, apply a 1.0 ml dose of test liquid to the pre-marked dosing location as follows. The tip of the pipette is held about 3 mm above the surface of the test specimen, and the dose is applied slowly (about 30 seconds) to avoid splashing. As soon as the entire dose has been applied, start a 1 minute timer. After 1 minute has elapsed, remove the test specimen and record the mass of the filter paper as Wet Mass$_{fp}$ to the nearest 0.0001 grams. Subtract the Dry Mass$_{fp}$ from the Wet Mass$_{fp}$ and record as z-Direction Leakage to the nearest 0.0001 grams.

In like fashion, repeat for a total of three replicate test specimens. Calculate the arithmetic mean for z-Direction Leakage and report to the nearest 0.0001 g.

Ordinary X-Y Plane Dimensions

For purposes herein, when a length or width of a feature of a pant is specified, it is to be measured with the pant laid out flat on a horizontal planar surface (in an opened or assembled configuration, as appropriate) with the material of the pant smoothed out flat, but in a relaxed condition, not pulled or stretched along any planar direction.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

While particular embodiments of the present invention have been illustrated and described, it would be obvious to those skilled in the art that various other changes and modifications can be made without departing from the scope of the invention. It is therefore intended to cover in the appended claims all such changes and modifications that are within the scope of this invention.

**Claims**

1. A durable absorbent pant adapted to accommodate one or more removable absorbent inserts, comprising:

   a front waist portion with a front waist edge and left and right front leg opening edges;
   a rear waist portion with a rear waist edge and left and right rear leg opening edges;
   a crotch portion comprising a gusset, the crotch portion having a forward portion meeting the front waist portion and a rearward portion meeting the rear waist portion; and
   left and right hip side portions joining the front waist portion to the rear waist portion and thereby forming a waist opening with a waist opening edge comprising the front waist edge and the rear waist edge, and left and right leg openings;
   wherein each of the front waist portion, rear waist portion and gusset comprises a knitted or woven material;
   wherein the gusset comprises a durable absorbent panel comprising a wearer-facing, liquid permeable top layer, a durable absorbent layer subjacent to the wearer-facing liquid permeable top layer, a liquid impermeable barrier layer subjacent to the durable absorbent layer, the wearer-facing liquid permeable top layer and the durable absorbent layer forming an enveloping structure having a closed end and an open end adapted to receive the one or more removable absorbent inserts in a position subjacent to the wearer-facing, liquid permeable top layer of the durable absorbent panel.

2. An absorbent system comprising the durable absorbent pant according to claim 1 and one or more absorbent inserts disposed within the enveloping structure, wherein each absorbent insert comprises an insert liquid impermeable backsheet and an insert absorbent core disposed above the insert liquid impermeable backsheet.

3. The absorbent system according to claim 2, wherein the insert absorbent core is positioned subjacent to the wearer-facing, liquid permeable top layer of the durable absorbent panel.

4. The absorbent system according to claim 2, wherein each of the one or more removable absorbent inserts further comprises an insert liquid permeable topsheet disposed above the insert absorbent core.

5. The absorbent system according to claim 4, wherein the insert liquid permeable topsheet is positioned subjacent to the wearer-facing, liquid permeable top layer of the durable absorbent panel.

6. The absorbent system according to any one of claims 2-5, wherein an outward-facing surface of the liquid impermeable backsheet of each of the one or more removable absorbent inserts is substantially free of adhesive.

7. The absorbent system according to any one of claims 2-6, wherein each of the one or more removable absorbent inserts comprises a pull tab.

8. The absorbent system according to claim 7, wherein the pull tab is hydrophobic.

9. The absorbent system according to any one of claims 2-8, wherein the one or more removable absorbent inserts comprises a first removable absorbent insert, a second removable absorbent insert, and, optionally, a third removable absorbent insert.

10. The absorbent system according to claim 9, wherein the first removable absorbent insert is positioned subjacent to the wearer-facing, liquid permeable top layer of the durable absorbent panel, the second removable absorbent insert is positioned subjacent to the first removable absorbent insert, and the optional third removable absorbent insert is positioned subjacent to the second removable absorbent insert.

11. The durable absorbent pant according to claim 1, wherein the open end of the enveloping structure comprises a slit in the wearer-facing, liquid permeable top layer of the durable absorbent panel.

12. The durable absorbent pant according to claim 11, wherein the slit is reinforced.

13. The durable absorbent pant according to claim 1, claim 11, or claim 12, wherein the durable absorbent panel comprises a liquid impermeable perimeter seal, wherein the open end of the enveloping structure is inboard of the liquid impermeable perimeter seal of the durable absorbent panel.

14. The absorbent system according to claim 2, wherein each of the one or more removable absorbent inserts has a bending stiffness in the machine direction (MD)/longitudinal of about 10 N/m$^2$ to about 100 N/m$^2$, preferably about 10 N/m$^2$ to about 80 N/m$^2$, more preferably about 20 N/m$^2$ to about 50 N/m$^2$.

15. The absorbent system according to any one of claims 2-10, wherein each of the one or more removable absorbent inserts has a bending stiffness in the cross direction (CD)/lateral of about 10 N/m$^2$ to about 100 N/m$^2$, preferably about 10 N/m$^2$ to about 80 N/m$^2$, more preferably about 20 N/m$^2$ to about 50 N/m$^2$.

**Patentansprüche**

1. Strapazierfähiges Absorptionshöschen, das konzipiert ist, um einen oder mehrere entfernbare Absorptionseinsätze zu fassen, umfassend:

einen vorderseitigen Taillenabschnitt mit einem vorderseitigen Taillenrand und einem linken und einem rechten vorderseitigen Beinöffnungsrand;
einen rückseitigen Taillenabschnitt mit einem rückseitigen Taillenrand und einem linken und einem rechten rückseitigen Beinöffnungsrand;
einen Schrittabschnitt, umfassend einen Zwickel, wobei der Schrittabschnitt einen vorwärts gerichteten Abschnitt, der auf den vorderseitigen Taillenabschnitt trifft, und einen rückwärtigen Abschnitt, der auf den rückseitigen Taillenabschnitt trifft, aufweist; und
einen linken und einen rechten Hüftenseitenabschnitt, die den vorderseitigen Taillenabschnitt mit dem rückseitigen Taillenabschnitt verbinden und wobei dadurch eine Taillenöffnung mit einem Taillenöffnungsrand, umfassend den vorderseitigen Taillenrand und den rückseitigen Taillenrand, und die linke und die rechte Beinöffnung ausgebildet werden;
wobei jeder des vorderseitigen Taillenabschnitts, des rückseitigen Taillenabschnitts und des Zwickels ein Strick- oder ein Gewebematerial umfasst;
wobei der Zwickel ein strapazierfähiges Absorptionsfeld umfasst, umfassend eine trägerseitige, flüssigkeitsdurchlässige obere Schicht, eine strapazierfähige Absorptionsschicht unterhalb der trägerseitigen, flüssigkeitsdurchlässigen oberen Schicht, eine flüssigkeitsundurchlässige Sperrschicht unterhalb der strapazierfähigen Absorptionsschicht, wobei die trägerseitige, flüssigkeitsdurchlässige obere Schicht und die strapazierfähige Absorptionsschicht eine umhüllende Konstruktion ausbilden, die ein geschlossenes Ende und ein offenes Ende aufweist, die konzipiert ist, um den einen oder die mehreren entfernbaren Absorptionseinsätze in einer Anordnung unterhalb der trägerseitigen, flüssigkeitsdurchlässigen oberen Schicht des strapazierfähigen Absorptionsfeldes aufzunehmen.

**2.** Absorptionssystem, umfassend das strapazierfähige Absorptionshöschen nach Anspruch 1 und einen oder mehrere Absorptionseinsätze, die innerhalb der umhüllenden Konstruktion eingerichtet sind, wobei jeder Absorptionseinsatz eine einsatzflüssigkeitsundurchlässige Unterschicht und einen Einsatzabsorptionskern umfasst, der oberhalb der einsatzflüssigkeitsundurchlässigen Unterschicht eingerichtet ist.

**3.** Absorptionssystem nach Anspruch 2, wobei der Einsatzabsorptionskern unterhalb der trägerseitigen, flüssigkeitsdurchlässigen oberen Schicht des strapazierfähigen Absorptionsfeldes angeordnet ist.

**4.** Absorptionssystem nach Anspruch 2, wobei jeder des einen oder der mehreren entfernbaren Absorptionseinsätze ferner eine einsatzflüssigkeitsdurchlässige Oberschicht umfasst, die oberhalb des Einsatzabsorptionskerns eingerichtet ist.

**5.** Absorptionssystem nach Anspruch 4, wobei die einsatzflüssigkeitsdurchlässige Oberschicht unterhalb der trägerseitigen, flüssigkeitsdurchlässigen oberen Schicht des strapazierfähigen Absorptionsfeldes angeordnet ist.

**6.** Absorptionssystem nach einem der Ansprüche 2 bis 5, wobei eine außenseitige Oberfläche der flüssigkeitsundurchlässigen Unterschicht jedes des einen oder der mehreren entfernbaren Absorptionseinsätze im Wesentlichen frei von Klebstoff ist.

**7.** Absorptionssystem nach einem der Ansprüche 2 bis 6, wobei jeder des einen oder der mehreren entfernbaren Absorptionseinsätze einen Pull-Tab umfasst.

**8.** Absorptionssystem nach Anspruch 7, wobei der Pull-Tab hydrophob ist.

**9.** Absorptionssystem nach einem der Ansprüche 2 bis 8, wobei der eine oder die mehreren entfernbaren Absorptionseinsätze einen ersten entfernbaren Absorptionseinsatz, einen zweiten entfernbaren Absorptionseinsatz und wahlweise einen dritten entfernbaren Absorptionseinsatz umfassen.

**10.** Absorptionssystem nach Anspruch 9, wobei der erste entfernbare Absorptionseinsatz unterhalb der trägerseitigen, flüssigkeitsdurchlässigen oberen Schicht des strapazierfähigen Absorptionsfeldes angeordnet ist, der zweite entfernbare Absorptionseinsatz unterhalb des ersten entfernbaren Absorptionseinsatzes angeordnet ist und der wahlweise dritte entfernbare Absorptionseinsatz unterhalb des zweiten entfernbaren Absorptionseinsatzes angeordnet ist.

**11.** Strapazierfähiges Absorptionshöschen nach Anspruch 1, wobei das offene Ende der umhüllenden Konstruktion einen Schlitz in der trägerseitigen, flüssigkeitsdurchlässigen oberen Schicht des strapazierfähigen Absorptionsfeldes umfasst.

**12.** Strapazierfähiges Absorptionshöschen nach Anspruch 11, wobei der Schlitz verstärkt ist.

**13.** Strapazierfähiges Absorptionshöschen nach Anspruch 1, 11 oder 12, wobei das strapazierfähige Absorptionsfeld eine flüssigkeitsundurchlässige Umfangsdichtung umfasst, wobei das offene Ende der umhüllenden Konstruktion innerhalb der flüssigkeitsundurchlässigen Umfangsdichtung des strapazierfähigen Absorptionsfeldes liegt.

**14.** Absorptionssystem nach Anspruch 2, wobei jeder des einen oder der mehreren entfernbaren Absorptionseinsätze eine Biegesteifigkeit in der Maschinenlaufrichtung (MD)/longitudinal von etwa 10 N/m$^2$ bis etwa 100 N/m$^2$, vorzugsweise etwa 10 N/m$^2$ bis etwa 80 N/m$^2$, mehr bevorzugt etwa 20 N/m$^2$ bis etwa 50 N/m$^2$ aufweist.

**15.** Absorptionssystem nach einem der Ansprüche 2 bis 10, wobei jeder des einen oder der mehreren entfernbaren Absorptionseinsätze eine Biegesteifigkeit in der Querrichtung (CD)/lateral von etwa 10 N/m$^2$ bis etwa 100 N/m$^2$, vorzugsweise etwa 10 N/m$^2$ bis etwa 80 N/m$^2$, mehr bevorzugt etwa 20 N/m$^2$ bis etwa 50 N/m$^2$ aufweist.

**Revendications**

**1.** Culotte absorbante durable conçue pour recevoir un ou plusieurs inserts absorbants amovibles, comprenant :

    une partie de taille avant avec un bord de taille avant et des bords d'ouverture de jambe avant gauche et droit ;

une partie de taille arrière avec un bord de taille arrière et des bords d'ouverture de jambe arrière gauche et droit ; une partie d'entrejambe comprenant un gousset, la partie d'entrejambe ayant une partie antérieure rencontrant la partie de taille avant et une partie postérieure rencontrant la partie de taille arrière ; et des parties latérales de hanche gauche et droite joignant la partie de taille avant à la partie de taille arrière et formant de ce fait une ouverture de taille avec un bord d'ouverture de taille comprenant le bord de taille avant et le bord de taille arrière, et des ouvertures de jambe gauche et droite ; dans laquelle chacun parmi la partie de taille avant, la partie de taille arrière et le gousset comprend un matériau tricoté ou tissé ; dans laquelle le gousset comprend un panneau absorbant durable comprenant une couche supérieure perméable aux liquides faisant face vers le porteur, une couche absorbante durable sous-jacente à la couche supérieure perméable aux liquides faisant face vers le porteur, une couche barrière imperméable aux liquides sous-jacente à la couche absorbante durable, la couche supérieure perméable aux liquides faisant face vers le porteur et la couche absorbante durable formant une structure d'enveloppement ayant une extrémité fermée et une extrémité ouverte conçue pour recevoir le ou les inserts absorbants amovibles dans une position sous-jacente à la couche supérieure perméable aux liquides faisant face vers le porteur du panneau absorbant durable.

2. Système absorbant comprenant la culotte absorbante durable selon la revendication 1 et un ou plusieurs inserts absorbants disposés au sein de la structure d'enveloppement, dans lequel chaque insert absorbant comprend une feuille de fond imperméable aux liquides d'insert et une âme absorbante d'insert disposée au-dessus de la feuille de fond imperméable aux liquides d'insert.

3. Système absorbant selon la revendication 2, dans lequel l'âme absorbante d'insert est positionnée sous-jacente à la couche supérieure perméable aux liquides faisant face vers le porteur du panneau absorbant durable.

4. Système absorbant selon la revendication 2, dans lequel chacun parmi le ou les inserts absorbants amovibles comprend en outre une feuille de dessus perméable aux liquides d'insert disposée au-dessus de l'âme absorbante d'insert.

5. Système absorbant selon la revendication 4, dans lequel la feuille de dessus perméable aux liquides d'insert est positionnée sous-jacente à la couche supérieure perméable aux liquides faisant face vers le porteur du panneau absorbant durable.

6. Système absorbant selon l'une quelconque des revendications 2 à 5, dans lequel une surface faisant face vers l'extérieur de la feuille de fond imperméable aux liquides de chacun parmi le ou les inserts absorbants amovibles est sensiblement dépourvue d'adhésif.

7. Système absorbant selon l'une quelconque des revendications 2 à 6, dans lequel chacun parmi le ou les inserts absorbants amovibles comprend une languette à tirer.

8. Système absorbant selon la revendication 7, dans lequel la languette à tirer est hydrophobe.

9. Système absorbant selon l'une quelconque des revendications 2 à 8, dans lequel le ou les inserts absorbants amovibles comprennent un premier insert absorbant amovible, un deuxième insert absorbant amovible et, facultativement, un troisième insert absorbant amovible.

10. Système absorbant selon la revendication 9, dans lequel le premier insert absorbant amovible est positionné sous-jacent à la couche supérieure perméable aux liquides faisant face vers le porteur du panneau absorbant durable, le deuxième insert absorbant amovible est positionné sous-jacent au premier insert absorbant amovible, et le troisième insert absorbant amovible facultatif est positionné sous-jacent au deuxième insert absorbant amovible.

11. Culotte absorbante durable selon la revendication 1, dans laquelle l'extrémité ouverte de la structure d'enveloppement comprend une fente dans la couche supérieure perméable aux liquides faisant face vers le porteur du panneau absorbant durable.

12. Culotte absorbante durable selon la revendication 11, dans laquelle la fente est renforcée.

13. Culotte absorbante durable selon la revendication 1, la revendication 11, ou la revendication 12, dans laquelle le panneau absorbant durable comprend un joint périmétrique imperméable aux liquides, dans laquelle l'extrémité ouverte de la structure d'enveloppement est à l'intérieur du joint périmétrique imperméable aux liquides du panneau absorbant durable.

14. Système absorbant selon la revendication 2, dans lequel chacun parmi le ou les inserts absorbants amovibles a une rigidité en flexion dans la direction machine (MD)/longitudinale d'environ 10 N/m$^2$ à environ 100 N/m$^2$, de préférence environ 10 N/m$^2$ à environ 80 N/m$^2$, plus préférablement environ 20 N/m$^2$ à environ 50 N/m$^2$.

15. Système absorbant selon l'une quelconque des revendications 2 à 10, dans lequel chacun parmi le ou les inserts absorbants amovibles a une rigidité en flexion dans la direction croisée (CD)/latérale d'environ 10 N/m$^2$ à environ 100 N/m$^2$, de préférence environ 10 N/m$^2$ à environ 80 N/m$^2$, plus préférablement environ 20 N/m$^2$ à environ 50 N/m$^2$.

FIG. 1

FIG. 2A

FIG. 2B

FIG. 3A    FIG. 3B    FIG. 3C    FIG. 3D    FIG. 3E

EP 4 577 168 B1

FIG. 4A

FIG. 4B

24

FIG. 5A

FIG. 5B

FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2021100698 A
- US 2021290447 A
- US 63315188
- EP 0861641 A2